(19) Europäisches Patentamt
European Patent Office
Office européen des brevets

(11) Veröffentlichungsnummer: **0 595 151 A2**

(12) # EUROPÄISCHE PATENTANMELDUNG

(21) Anmeldenummer: **93116789.4**

(22) Anmeldetag: **18.10.93**

(51) Int. Cl.[5]: **C07D 471/04**, A61K 31/435, //(C07D471/04,235:00,221:00)

(30) Priorität: **24.10.92 DE 4236026**

(43) Veröffentlichungstag der Anmeldung: **04.05.94 Patentblatt 94/18**

(84) Benannte Vertragsstaaten: **AT BE CH DE DK ES FR GB GR IE IT LI LU NL PT SE**

(71) Anmelder: **MERCK PATENT GmbH**
**Frankfurter Strasse 250**
**D-64293 Darmstadt(DE)**

(72) Erfinder: **Mederski, Werner, Dr.**
**Am Ohlenberg 29**
**D-64390 Erzhausen(DE)**
Erfinder: **Dorsch, Dieter, Dr.**
**Königsberger Strasse 17A**
**D-64372 Ober-Ramstadt(DE)**
Erfinder: **Bathe, Andreas, Dr.**
**August-Bebel-Strasse 59**
**D-64347 Griesheim(DE)**
Erfinder: **Hartig, Thorsten, Dr.**
**Am Schulacker 9-13**
**D-64846 Gross-Zimmern(DE)**
Erfinder: **Osswald, Mathias, Dr.**
**Katzenelnbogenweg 1**
**D-64673 Zwingenberg(DE)**
Erfinder: **Beier, Norbert, Dr.**
**Georgenhäuser-Strasse 19**
**D-64354 Reinheim 5(DE)**
Erfinder: **Schelling, Pierre, Prof. Dr.**
**Bordenbergweg 17**
**D-64367 Mühltal(DE)**
Erfinder: **Minck, Klaus-Otto, Dr.**
**Büchestrasse 8**
**D-64372 Ober-Ramstadt(DE)**
Erfinder: **Lues, Ingeborg, Dr.**
**Katharinenstrasse 2**
**D-64297 Darmstadt(DE)**

(54) **Imidazopyridinderivate als Angiotensin II Antagonisten.**

(57) Neue Imidazopyridinderivate der Formel I

EP 0 595 151 A2

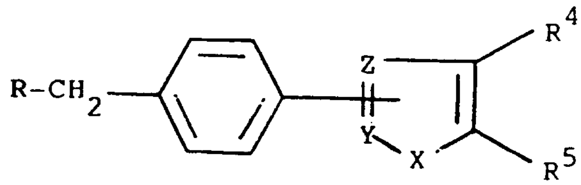

I

worin
R

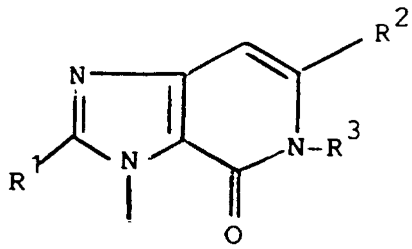

bedeutet und X, -Y=Z-, $R^1$, $R^2$, $R^3$, $R^4$ und $R^5$ die in Patentanspruch 1 angegebenen Bedeutungen haben, sowie deren Salze zeigen Angiotensin II-antagonistische Eigenschaften und können zur Behandlung von Hypertension, Aldosteronismus, Herzinsuffizienz und erhöhtem Augeninnendruck sowie von Störungen des Zentralnervensystems verwendet werden.

Die Erfindung betrifft neue Imidazopyridinderivate der Formel I

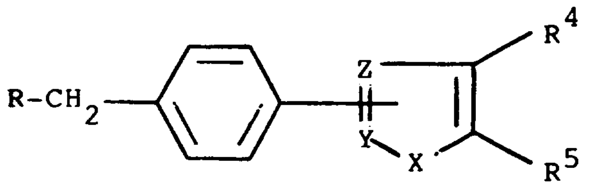

I

worin

R

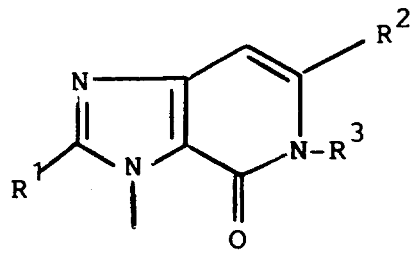

,

| | |
|---|---|
| X | O, S oder $NR^6$, |
| -Y=Z- | $-CR^7=C-$, $-C=CR^7-$, $-N=C-$ oder $-C=N-$, |
| $R^1$ | A, Alkenyl oder Alkinyl mit jeweils bis zu 6 C-Atomen, Cycloalkyl mit 3-7 C-Atomen, OA oder SA, |
| $R^2$ | H oder Hal, |
| $R^3$ | H, $R^8$ oder $C_nH_{2n}-R^9$, |
| $R^4$ und $R^5$ | jeweils H, A oder Hal, |
| $R^6$ | H oder $-C_mH_{2m}-R^{10}$, |
| $R^7$ und $R^{10}$ | jeweils CN, $COOR^{11}$ oder 1H-5-Tetrazolyl, |
| $R^8$ | Alkyl mit 1-6 C-Atomen, worin auch ein oder mehrere H-Atom(e) durch F ersetzt sein kann (können), |
| $R^9$ | $COOR^{12}$, $CONR^{12}R^{13}$, COA, $NR^{12}R^{13}$, Cycloalkyl mit 3-7 C-Atomen, Ar, Het, COAr oder COHet, |
| $R^{11}$, $R^{12}$ und $R^{13}$ | jeweils H, A oder Ar, |
| A | Alkyl mit 1-6 C-Atomen, |
| Ar | eine unsubstituierte oder eine durch $R^8$, OH, $OR^8$, COOH, COOA, CN, $NO_2$, $NH_2$, $NHCOR^8$, $NHSO_2R^8$, Hal oder 1H-5-Tetrazolyl mono- oder disubstituierte Phenylgruppe, |
| Het | einen fünf- oder sechsgliedrigen heteroaromatischen Rest mit 1 bis 3 N-, O- und/oder S-Atomen, der auch ein- oder zweifach durch A substituiert und/oder mit einem Benzol- oder Pyridinring kondensiert sein kann, |
| Hal | F, Cl, Br oder I und |
| m und n | jeweils 1, 2, 3, 4, 5 oder 6 bedeuten, |

sowie ihre Salze.

Ähnliche Verbindungen sind aus der EP-A2-0400 974 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie angiotensin II-antagonistische Eigenschaften und können daher zur Behandlung der angiotensin II-abhängigen Hypertension, des Aldosteronismus, der Herzinsuffizienz und des erhöhten Augeninnendrucks sowie von Störungen des Zentralnervensystems eingesetzt werden.

Diese Wirkungen können nach üblichen in vitro- oder in vivo-Methoden ermittelt werden, wie sie z.B. in der US-PS 4 880 804, der US-PS 5 036 048 und der WO 91/14367 beschrieben sind, ferner von A.T. Chiu et al., J. Pharmacol. Exp. Therap. 250, 867-874 (1989), und von P.C. Wong et al., ibid. 252, 719-725 (1990; in vivo, an Ratten).

Die Verbindungen der Formel I können als Arzneimittelwirkstoffe in der Human- und Veterinärmedizin eingesetzt werden, insbesondere zur Prophylaxe und/oder Therapie von Herz-, Kreislauf- und Gefäßkrankheiten, vor allem von Hypertonie, Herzinsuffizienz und Hyperaldosteronismus, ferner von Hypertrophie und Hyperplasie der Blutgefäße und des Herzens, Angina pectoris, Herzinfarkten, Schlaganfall, Restenosen nach Angioplastie oder By-pass-Operationen, von ischämischen peripheren Durchblutungsstörungen, Arteriosklerose, erhöhtem Augeninnendruck, Glaukomen, macularer Degeneration, Hyperurikämie, Nierenfunktionsstörungen, z.B. Nierenversagen, Nephropathia diabetica, Retinopathia diabetica, Psoriasis, von gastrointestinalen Erkrankunen, Blasenerkrankungen, Lungenödemen, chronischer Bronchitis, angiotensin II-vermittelten Störungen in weiblichen Fortpflanzungsorganen, Wanrnehmungsstörungen, z.B. Demenz, Amnesie, Gedächnisfunktionsstörungen, Angstzuständen, Depression, Epilepsie, des Parkinson-Syndroms oder der Bulimie.

Gegenstand der Erfindung sind die Verbindungen der Formel I und ihre Salze sowie ein Verfahren zur Herstellung dieser Verbindungen, dadurch gekennzeichnet, daß man

(a) eine Verbindung der Formel II

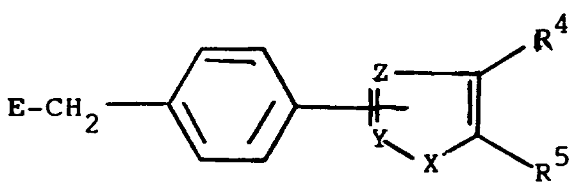

II

worin

E Cl, Br, I oder eine freie oder reaktionsfähig funktionell angewandelte OH-Gruppe bedeutet und

X, -Y=Z-, $R^4$ und $R^5$ die in Anspruch 1 angegebene Bedeutung haben,

mit einer Verbindung der Formel III

H-R III

worin

R die in Anspruch 1 angegebene Bedeutung hat,

umsetzt

oder

(b) eine Verbindung der Formel IV

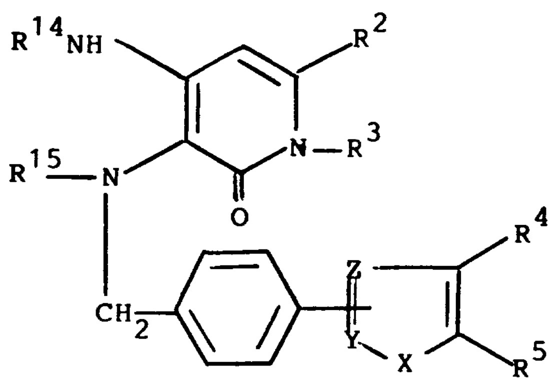

IV

worin

$R^{14}$ $R^1$-CO oder H und

$R^{15}$ H (falls $R^{14}$ $R^1$-CO ist) oder $R^1$-CO (falls $R^{14}$ H ist)

bedeuten

und

X, -Y = Z-, $R^1$, $R^2$, $R^3$, $R^4$ und $R^5$ die in Anspruch 1 angegebenen Bedeutungen haben,

mit einem cyclisierenden Mittel behandelt,

oder

(c) eine Verbindung der Formel I aus einem ihrer funktionellen Derivate durch Behandeln mit einem solvolysierenden oder hydrogenolysierenden Mittel in Freiheit setzt,

und/oder daß man in einer Verbindung der Formel I einen oder mehrere Rest(e) R, X und/oder -Y = Z- in einen oder mehrere andere Reste R, X und/oder -Y = Z- umwandelt und/oder eine Base oder Säure der Formel I in eines ihrer Salze umwandelt.

Vor- und nachstehend haben die Reste bzw. Parameter R, $R^1$ bis $R^{15}$, X, -Y = Z-, A, Ar, Het, Hal, m, n und E die bei den Formeln I bis IV angegebenen Bedeutungen, falls nicht ausdrücklich etwas anderes angegeben ist.

In den vorstehenden Formeln hat A 1-6, vorzugsweise 1, 2, 3 oder 4 C-Atome. A bedeutet vorzugsweise Methyl, weiterhin Ethyl, Propyl, Isopropyl, Butyl, Isobutyl, sek.-Butyl, oder tert.-Butyl, ferner auch Pentyl. 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methoxypropyl, 1,1,2- oder 1,2,2-Trimethylpropyl. Alkenyl steht vorzugsweise für Vinyl, 1- oder 2-Propenyl, 1-Butenyl, ferner 1-Pentenyl oder 1-Hexenyl. Alkinyl steht vorzugsweise für Ethinyl, 1- oder 2-Propinyl, ferner 1-Butinyl, 1-Pentinyl oder 1-Hexinyl. Cycloalkyl bedeutet vorzugsweise Cyclopropyl, ferner Cyclobutyl, Cyclopentyl, Cyclohexyl oder Cycloheptyl. Der Rest OA ist vorzugsweise Methoxy, Ethoxy oder Propoxy. Der Rest SA ist vorzugsweise Methylthio, Ethylthio oder Propylthio. Falls mehrere Reste A oder Cycloalkyl in einer Verbindung der Formel I vorhanden sind, so können sie gleich oder voneinander verschieden sein.

Hal bedeutet vorzugsweise F, Cl oder Br, aber auch J.

R ist ein von 3H-Imidazo[4,5-c]pyridin ("3H-IP") abgeleiteter Rest, genauer 2-$R^1$-4-oxo-5-$R^3$-6-$R^2$-4,5-dihydro-3H-imidazo[4,5-c]pyridin-3-yl.

Ar ist vorzugsweise unsubstituiertes, ferner - wie angegeben - monosubstituiertes Phenyl, im einzelnen bevorzugt Phenyl, o-, m- oder p-Tolyl, o-, m- oder p-Ethylphenyl, o-, m- oder p-Trifluormethylphenyl, o-, m- oder p-Hydroxyphenyl, o-, m- oder p-Methoxyphenyl, o-, m- oder p-Ethoxyphenyl, o-, m- oder p-(Difluormethoxy)-phenyl, o-, m- oder p-(Trifluormethoxy)-phenyl, o-, m- oder p-Carboxyphenyl, o-, m- oder p-Methoxycarbonylphenyl, o-, m- oder p-Ethoxycarbonylphenyl, o-, m- oder p-Cyanphenyl, o-, m- oder p-Nitrophenyl, o-, m- oder p-Aminophenyl, o-, m- oder p-Acetamidophenyl, o-, m- oder p-Trifluoracetamidophenyl, o-, m- oder p-Methylsulfonamidophenyl, o-, m- oder p-Trifluormethylsulfonamidophenyl, o-, m- oder p-Fluorphenyl, o-, m- oder p-Chlorphenyl, o-, m- oder p-Bromphenyl, o-, m- oder p-(1H-5-Tetrazolyl)-phenyl, ferner bevorzugt 2,3-, 2,4-, 2,5-, 2,6-, 3,4- oder 3,5-Dimethylphenyl, 2,3-, 2,4-, 2,5-, 2,6-, 3,4- oder 3,5-Dimethoxyphenyl.

Het ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt

1,2,3-Triazol-1-, -4- oder -5-yl, 1,2,4-Triazol-1-, -3- oder -5-yl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -4-yl, 2,1,5-Thiadiazol-3- oder -4-yl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2-1, 3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolyl, 2-, 4-, 5-, 6-, 7- oder 8-Chinazolyl,

1H-1-, -2-, -5-, -6- oder -7-Imidazo[4,5-b]pyridyl, 3H-2-, -3-, -5-, -6- oder -7-Imidazo[4,5-b]pyridyl, 1H-1-, -2-, -4-, -6- oder -7-Imidazo[4,5-c]pyridyl, 3H-2-, -3-, -4-, -6- oder -7-Imidazo[4,5-c]pyridyl.

In den Begriff "Het" eingeschlossen sind auch die homologen Reste, in denen der heteroaromatische Ring durch eine oder mehrere, vorzugsweise 1 oder 2, A-Gruppen, vorzugsweise Methyl- und/oder Ethylgruppen substituiert ist, z.B. 3-, 4- oder 5-Methyl-2-furyl, 2-, 4- oder 5-Methyl-3-furyl, 2,4-Dimethyl-3-furyl, 3-, 4- oder 5-Methyl-2-thienyl, 3-Methyl-5-tert.-butyl-2-thienyl,2-, 4- oder 5-Methyl-3-thienyl, 2- oder 3-Methyl-1-pyrrolyl, 1-, 3-, 4- oder 5-Methyl-2-pyrrolyl, 3,5-Dimethyl-4-ethyl-2-pyrrolyl, 2-, 4- oder 5-Methyl-1-imidazolyl,4-Methyl-5-pyrazolyl, 4- oder 5-Methyl-3-isooxazolyl, 3- oder 5-Methyl-4-isoxazolyl, 3- oder 4-Methyl-5-isoxazolyl, 3,4-Dimethyl-5-isoxazolyl, 4- oder 5-Methyl-2-thiazolyl, 4- oder 5-Ethyl-2-thiazolyl, 2- oder 5-Methyl-4-thiazolyl, 2- oder 4-Methyl-5-thiazolyl, 2,4-Dimethyl-5-thiazolyl, 3-, 4-, 5- oder 6-Methyl-2-pyridyl, 2-, 4-, 5- oder 6-Methyl-3-pyridyl, 2- oder 3-Methyl-4-pyridyl, 4-Methyl-2-pyrimidinyl, 4,5-Dimethyl-2-pyrimidinyl, 2-, 5- oder 6-Methyl-4-pyrimidinyl, 2,6-Dimethyl-4-pyrimidinyl, 3-, 4-, 5-, 6- oder 7-Methyl-2-benzofuryl, 2-Ethyl-3-benzofuryl, 3-, 4-, 5-, 6- oder 7-Methyl-2-benzothienyl, 3-Ethyl-2-benzothienyl, 1-, 2-, 4-, 5-, 6- oder 7-Methyl-3-indolyl, 1-Methyl-5- oder 6-benzimidazolyl, 1-Ethyl-5- oder 6-benzimidazolyl.

Der Rest $R^1$ ist vorzugsweise geradkettig und steht bevorzugt für A, Alkenyl oder Cycloalkyl mit jeweils 3-6 C-Atomen, insbesondere Butyl, ferner Propyl, Pentyl, Hexyl, Allyl, 1-Propenyl, Cyclopropyl, ferner 1-Butenyl, 1-Pentenyl, 1-Hexenyl, 1-Propinyl, 1-Butinyl, 1-Pentinyl, 1-Hexinyl, Cyclobutyl oder Cyclopentyl.

Der Rest $R^2$ ist vorzugsweise H, aber auch F, Cl, Br oder I.

Der Rest $R^3$ steht bevorzugt für $-C_nH_{2n}R^9$ (im einzelnen bevorzugt für $-CH_2R^9$).

Die Reste $R^4$ und $R^5$ sind bevorzugt gleich und bedeuten vorzugsweise H, aber auch F, Cl, Br oder I.

Der Rest $R^6$ ist vorzugsweise H oder $CH_2$-$R^{10}$.

Der Rest $R^7$ ist vorzugsweise CN oder 1H-5-Tetrazolyl.

Der Rest $R^8$ enthält bevorzugt 1, 2 oder 3 C-Atome und bedeutet vorzugsweise Methyl, Ethyl, Trifluormethyl, Pentafluorethyl, 2,2,2-Trifluorethyl oder 3,3,3-Trifluorpropyl. Falls eine Verbindung der Formel I zwei Reste $R^8$ enthält, so können diese gleich oder voneinander verschieden sein.

Der Rest $R^9$ ist vorzugsweise COOH; COOA, insbesondere $COOCH_3$ oder $COOC_2H_5$; CONHA; insbesondere $CONHCH_3$ oder $CONHC_2H_5$; $CON(A)_2$, insbesondere $CON(CH_3)_2$ oder $CON(C_2H_5)_2$; CONHAr, insbesondere $CONHC_6H_5$ oder CONH-(2,6-dimethylphenyl); COA, insbesondere $COCH_3$, $COC_2H_5$, $COC_3H_7$, $COCH(CH_3)_2$ oder $COC(CH_3)_3$; COAr, insbesondere $COC_6H_5$, CO-(2-$CH_3O$-$C_6H_4$) oder CO-(2-$CH_3$-$C_6H_4$).

$R^{10}$ ist vorzugsweise COOH oder COOA.

$R^{11}$, $R^{12}$ und $R^{13}$ sind jeweils vorzugsweise H, $CH_3$ oder $C_2H_5$.

m ist vorzugsweise 1, ferner bevorzugt 2.

n ist vorzugsweise 1, ferner bevorzugt 2, 3 oder 4.

Die Gruppe $-C_mH_{2m}-$ steht insbesondere für $-(CH_2)_m-$, bevorzugt für $-CH_2-$. Die Gruppe $-C_nH_{2n}$ steht insbesondere für $-(CH_2)_n-$, bevorzugt für $-CH_2-$.

Der Rest X ist vorzugsweise S, ferner bevorzugt $NR^6$. Die Gruppe -Y=Z- ist vorzugsweise

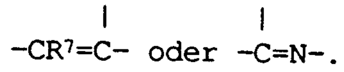

Die Verbindungen der Formel I können ein odere mehrere chirale Zentren besitzen und daher in verschiedenen - optisch-aktiven oder optisch-inaktiven - Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ih ausgedrückt werden, die der Formel I entsprechen und worin die nicht näher bezeichneten Reste die bei

Formel I angegebenen Bedeutungen haben, worin jedoch die Gruppe

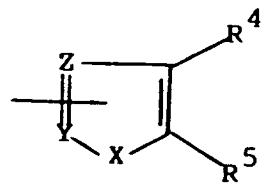

in Ia 2-Cyan-3-thienyl bedeutet;
in Ib 2-(1H-5-Tetrazolyl)-3-thienyl bedeutet;
in Ic 1-Cyanmethyl-2-imidazolyl bedeutet;
in Id 1-Carboxymethyl-2-imidazolyl bedeutet;
in Ie 1-(1H-5-Tetrazolyl)-2-imidazolyl bedeutet;
in If 1-Cyanmethyl-4,5-dichlor-2-imidazolyl bedeutet;
in Ig 1-Carboxymethyl-4,5-dichlor-2-imidazolyl bedeutet;
in Ih 1-(1H-5-Tetrazolyl)-4,5-dichlor-2-imidazolyl bedeutet.

Verbindungen der Formeln Ia und Ib sind besonders bevorzugt.

Weiterhin sind bevorzugt:

Verbindungen der Formeln Ii sowie Iai bis Ihi, die den Verbindungen der Formeln I sowie Ia bis Ih entsprechen, worin jedoch zusätzlich $R^1$ A oder Cycloalkyl mit 3-7 C-Atomen, insbesondere jedoch Butyl bedeutet;

Verbindungen der Formeln Ij, Iaj bis Iij sowie Iaij bis Ihij, die den Formeln I, Ia bis Ii sowie Iai bis Ihi entsprechen, worin jedoch zusätzlich $R^2$ H bedeutet;

Verbindungen der Formeln Ik sowie Iak bis Ihk, die den Formeln I sowie Ia bis Ih entsprechen, worin jedoch zusätzlich

$R^1$ Butyl und
$R^2$ H bedeutet.

Weitere bevorzugte Gruppen von Verbindungen entsprechen der Formel I sowie den anderen vorstehend genannten Formeln, worin jedoch der Rest $R^3$ folgende Bedeutungen hat:

(a) H,
(b) $R^8$,
(c) A,
(d) $-C_nH_{2n}-R^9$,
(e) $-CH_2-R^9$,
(f) $-COOR^{12}$,
(g) $-CONR^{12}R^{13}$,
(h) -COA,
(i) $-NR^{12}R^{13}$,
(j) $-(C_3-C_7$-Cycloalkyl),
(k) $-CH_2$-Ar,
(l) $-CH_2$-Het,
(m) $-CH_2$-COAr,
(n) $-CH_2$-COHet,
(o) unsubstituiertes oder (vorzugsweise in 2-Stellung) durch F, Cl, $COOR^{12}$, $NO_2$, $NH_2$, $N(A)_2$ oder NHCOA einfach substituiertes Benzyl,
(p) A oder $-CH_2-R^9$, wobei $R^9$ COOH, COOA, $CON(A)_2$, $CONHC_6H_5$, CONH(2,6-di-$CH_3$-$C_6H_3$), COA, $C_6H_5$, eine in 2-Stellung durch F, Cl, COOH, COOA, $NO_2$, $NH_2$, $N(A)_2$ oder NHCO einfach substituierte Benzylgruppe, Benzoyl oder 2-Methoxybenzoyl bedeutet,
(q) 2-(COOA)-benzyl.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; insbesondere aber in

der EP-A2-O 430 709 und in der US-PS 4 880 804) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können vorzugsweise erhalten werden, indem man Verbindungen der Formel II mit Verbindungen der Formel III umsetzt.

In den Verbindungen der Formel II bedeutet E vorzugsweise Cl, Br, I oder eine reaktionsfähig funktionell abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl- oder P-Tolylsulfonyloxy).

Die Umsetzung von (II mit III erfolgt zweckmäßig, indem man zunächst III durch Behandeln mit einer Base in ein Salz umwandelt, z.B. mit einem Alkalimetallalkoholat wie $CH_3ONa$ oder K-tert.-Butylat in einem Alkohol wie Methanol oder tert.-Butanol oder mit einem Alkalimetallhydrid wie NaH oder einem Alkalimetallalkoholat in Dimethylformamid (DMF), und dieses dann in einem inerten Lösungsmittel, z.B. einem Amid wie DMF oder Dimethylacetamid oder einem Sulfoxid wie Dimethylsulfoxid (DMSO), mit II umsetzt, zweckmäßig bei Temperaturen zwischen -20 und 100°, vorzugsweise zwischen 10 und 30°. Als Basen eignen sich auch Alkalimetall-hydrogencarbonate wie $NaHCO_3$ oder $KHCO_3$.

Die Verbindungen der Formel I sind weiterhin durch Cyclisierung von Verbindungen der Formel IV erhältlich. Diese Cyclisierung gelingt zweckmäßig durch Erhitzen mit Polyphosphorsäure, Essigsäure oder Diglyme auf Temperaturen zwischen etwa 80 und 180°, vorzugsweise zwischen 120 und 160°.

Weiterhin kann man eine Verbindung der Formel I durch Solvolyse (z.B. Hydrolyse) oder Hydrogenolyse aus einem ihrer funktionellen Derivate in Freiheit setzen.

So ist es möglich, nach einer der angegebenen Methoden eine Verbindung herzustellen, die der Formel I entspricht, aber an Stelle einer 5-Tetrazolylgruppe eine in 1-Stellung (oder 2-Stellung) funktionell abgewandelte (durch eine Schutzgruppe geschützte) 1H-(oder 2H)-5-Tetrazolylgruppe enthält. Als Schutzgruppe eignen sich beispielsweise: Triphenylmethyl, abspaltbar mit HCl oder Ameisensäure in einem inerten Lösungsmittel oder Lösungsmittelgemisch, z.B. Dioxan oder Ether/Dichlormethan/Methanol; 2-Cyanethyl, abspaltbar mit NaOH in Wasser/THF; p-Nitrobenzyl, abspaltbar mit $H_2$/Raney-Nickel in Ethanol (vgl. EP-A2-O 291 969).

Die Ausgangsstoffe, insbesondere diejenigen der Formel II, sind teilweise bekannt. Falls sie nicht bekannt sind, können sie nach bekannten Methoden in Analogie zu bekannten Stoffen hergestellt werden.

Verbindungen der Formel III sind z.B. erhältlich durch Reaktion von Carbonsäuren der Formel $R^1$-COOH mit Verbindungen der Formel V

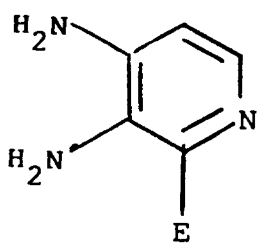

V

in Gegenwart von Polyphosphorsäure; dabei wird die Gruppe E (vorzugsweise Cl) hydrolysiert, und es entstehen zunächst Verbindungen entsprechend Formel III mit $R^3$ = H, die, falls erwünscht, anschließend mit Verbindungen der Formel E-$R^3$ (worin $R^3$ von H verschieden ist) umgesetzt werden können.

Verbindungen der Formel IV sind z.B. erhältlich durch Umsetzung von Verbindungen der Formel VI

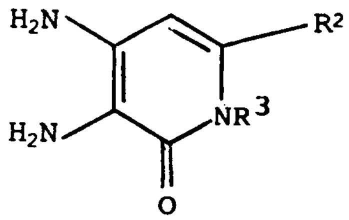

VI

worin jedoch die eine Aminogruppe durch eine Aminoschutzgruppe (z.B. Benzyl, A-O-CO- oder Benzyloxycarbonyl) geschützt ist, mit Verbindungen der Formel II sowie nachfolgende Abspaltung der Schutzgruppe und Reaktion mit Säuren der Formel $R^1$-COOH oder deren funktionellen Derivaten; sie werden in der Regel nicht isoliert, sondern entstehen in situ bei der letztgenannten Umsetzung.

Es ist ferner möglich, eine Verbindung der Formel I in eine andere Verbindung der Formel I umzuwandeln, indem man einen oder mehrere der Reste R, X und/oder -Y=Z- in andere Reste R, X und/oder -Y=Z- umwandelt, z.B. indem man Nitrogruppen (beispielsweise durch Hydrierung an Raney-Nickel oder Pd-Kohle in einem inerten Lösungsmittel wie Methanol oder Ethanol) zu Aminogruppen reduziert und/oder freie Amino- und/oder Hydroxygruppen funktionell abwandelt und/oder funktionell abgewandelte Amino- und/oder Hydroxygruppen durch Solvolyse oder Hydrogenolyse freisetzt und/oder Nitrilgruppen zu COOH-Gruppen hydrolysiert oder mit Derivaten der Stickstoffwasserstoffsäure, z.B. Natriumazid in N-Methylpyrrolidon oder Trimethylzinnazid in Toluol, zu Tetrazolylgruppen umsetzt.

So kann man beispielsweise freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und/oder in Gegenwart einer Base wie Triethylamin oder Pyridin bei Temperaturen zwischen -60 und +30°.

Umgekehrt kann in einer Verbindung der Formel I eine funktionell abgewandelte Amino- und/oder Hydroxygruppe durch Solvolyse oder Hydrogenolyse nach üblichen Methoden in Freiheit gesetzt werden. So kann z.B. eine $NHCOR^8$- oder eine COOA-Gruppe in die entsprechende $NH_2$- oder HOOC-Gruppe umgewandelt werden. COOA-Gruppen können z.B. mit NaOH oder KOH in Wasser, Wasser-THF oder Wasser-Dioxan bei Temperaturen zwischen 0 und 100° verseift werden.

Umsetzung von Nitrilen der Formel I (z.B. solche mit $R^7$ oder $R^{10}$ = CN) mit Derivaten der Stickstoffwasserstoffsäure führt zu Tetrazolen der Formel I (z.B. mit $R^7$ oder $R^{10}$ = 1H-5-Tetrazolyl). Bevorzugt verwendet man Trialkylzinnazide wie Trimethylzinnazid in einem inerten Lösungsmittel, z.B. einem aromatischen Kohlenwasserstoff wie Toluol bei Temperaturen zwischen 20 und 150°, vorzugsweise zwischen 80 und 140°, oder Natriumazid in N-Methylpyrrolidon bei Temperaturen zwischen etwa 100 und 200°. Anschließend wird die Trialkylzinn-Gruppe abgespalten, entweder durch Behandeln mit Salzsäure, z.B. in Dioxan, oder mit Alkali, z.B. in Ethanol/Wasser, oder mit Ameisensäure z.B. in Methanol, oder durch Chromatographie an einer Kieselgel-Säule, z.B. mit Ethylacetat/Methanol.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Apfelsäure, Citronensäure, Gluconsäure, Ascorbinsäure, Nicotinsäure, Isonicotinsäure, Methan- oder Ethansulfonsäure, Ethandisulfonsäure; 2-Hydroxyethansulfonsäure, Benzolsulfonsäure, p-Toluolsulfonsäure, Naphthalinmono- und disulfonsäuren, Laurylschwefelsäure. Salze mit physiologisch nicht unbedenklichen Säuren, z.B. Pikrate, können zur Isolierung und/oder Aufreinigung der Verbindung der Formel I verwendet werden.

Andererseits können Verbindungen der Formel I, die COOH- und/oder Tetrazolylgruppen enthalten, mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden. Die Kaliumsalze der Tetrazolylderivate sind besonders bevorzugt.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff-(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind spezielle Lacktabletten und Kapseln mit magensaftresistenten Überzügen bzw. Kapselhüllen. Zur rektalen Anwendung dienen-Suppositorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z.B. Fluorchlorkohlenwaserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine, Diuretika, Antiphlogistika.

Die erfindungsgemäßen Substanzen werden in der Regel in Analogie zu anderen bekannten, im Handel befindlichen Präparaten, insbesondere aber in Analogie zu den in der US-PS 4 880 804 beschriebenen Verbindungen verabreicht, vorzugsweise in Dosierungen zwischen etwa 1 mg und 1 g, insbesondere zwichen 50 und 500 mg pro Dosierungseinheit. Die tägliche Dosierung liegt vorzugsweise zwischen etwa 0,1 und 50 mg/kg, insbesondere 1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und/oder durch Kristallisation.
IP = Imidazo[4,5-c]pyridin, IPe = Imidazo[4,5-c]pyridine.

Beispiel 1

Eine Lösung von 0,23 g Na in 20 ml Methanol wird innerhalb 15 Minuten zugetropft zu einer Lösung von 1,91 g 2-Butyl-4,5-dihydro-4-oxo-1(oder 3)H-IP ("IIIa"; erhältlich durch Kondensation von Valeriansäure mit 3,4-Diamino-2-chlorpyridin in Gegenwart von Polyphosphorsäure) in 75 ml Methanol. Man rührt noch 30 Minuten bei 20°, dampft ein, löst den Rückstand in 20 ml DMF und tropft bei 0° unter Rühren eine Lösung von 2,78 g 2-Cyan-3-(4-brommethylphenyl)-thiophen ("IIA"; F. 58°; erhältlich durch Reaktion von 2-Cyan-3-bromthiophen mit 4-(Dimethyl-(1,1,2-trimethylpropyl)-silyloxy-methyl)-phenylboronsäure zu 2-Cyan-3-(4-(dimethyl-(1,1,2-trimethylpropyl)-silyloxy-methyl)-phenyl)-thiophen und Umsetzung mit Triphenylphosphinbromid) in 10 ml DMF hinzu. Man rührt 16 Stunden bei 20°, dampft ein, arbeitet wie üblich auf, chromatographiert an Kieselgel und erhält 2-Butyl-3-(4-(2-cyan-3-thienyl)-benzyl)-4,5-dihydro-4-oxo-3H-IP, F. 219 °.

Analog erhält man aus IIIa:

mit 2-Methoxycarbonyl-3-(4-brommethylphenyl)-thiophen (erhältlich aus 3-Bromthiophen-2-carbonsäuremethylester über 2-Methoxycarbonyl-3-(4-(dimethyl(1,1,2-trimethylpropyl)-silyloxy-methyl)-phenyl)-thiophen analog IIa):

2-Butyl-3-(4-(2-methoxycarbonyl-3-thienyl)-benzyl)-4,5-dihydro-4-oxo-3H-IP;

mit 1-Cyanmethyl-2-(4-brommethylphenyl)-imidazol (erhältlich aus 1-Cyanmethyl-2-brom-imidazol analog IIa):

2-Butyl-3-(4-(1-cyanmethyl-2-imidazolyl)-benzyl)-4,5-dihydro-4-oxo-3H-IP;

mit 1-Cyanmethyl-2-(4-brommethylphenyl)-4,5-dichlor-imidazol (erhältlich aus 1-Cyanmethyl-2-brom-4,5-dichlor-imidazol analog IIa):

2-Butyl-3-(4-(1-cyanmethyl-4,5-dichlor-2-imidazolyl)-benzyl)-4,5-dihydro-4-oxo-3H-IP;

mit 1-Ethoxycarbonylmethyl-2-(4-brommethylphenyl)-imidazol (erhältlich aus 1-Ethoxycarbonylmethyl-2-brom-imidazol analog IIa):

2-Butyl-3-(4-(1-ethoxycarbonylmethyl-2-imidazolyl)-benzyl)-4,5-dihydro-4-oxo-3H-IP;

mit 1-Ethoxycarbonylmethyl-2-(4-brommethylphenyl)-4,5-dichlor-imidazol (F. 99-100°; erhältlich aus 1-Ethoxycarbonylmethyl-2-brom-4,5-dichlor-imidazol analog IIa):

2-Butyl-3-(4-(1-ethoxycarbonylmethyl-4,5-dichlor-2-imidazolyl)-benzyl)-4,5-dihydro-4-oxo-3H-IP.

Analog erhält man aus IIa:

mit 2-Propyl-4,5-dihydro-4-oxo-1(oder 3)H-IP:

2-Propyl-3-(4-(2-cyan-3-thienyl)-benzyl)-4,5-dihydro-4-oxo-3H-IP;

mit 2-Cyclopropyl-4,5-dihydro-4-oxo-1(oder 3)H-IP:

2-Cyclopropyl-3-(4-(2-cyan-3-thienyl)-benzyl)-4,5-dihydro-4-oxo-3H-IP.

Beispiel 2

Ein Gemisch von 1,02 g Valeriansäure, 4,50 g 4-Amino-1,2-dihydro-2-oxo-3-(4-(2-(1H-5-tetrazolyl)-3-thienyl)-benzylamino)-1-(N,N-dimethylcarbamoylmethyl)-pyridin [erhältlich durch Reaktion von 3-Amino-4-benzylamino-1,2-dihydro-2-oxo-1-(N,N-dimethylcarbamoylmethyl)-pyridin mit IIa zu 4-Benzylamino-3-oxo-1-(N,N-dimethylcarbamoylmethyl)-pyridin,Reaktion mit Trimethylzinnazid zu 4-Benzylamino-1,2-dihydro-2-oxo-3-(4-(2-(1H-5-tetrazolyl)-3-thienyl)-benzyl-amino)-1-(N,N-dimethylcarbamoylmethyl)-pyridin und hydrogenolytische Abspaltung der Benzylgruppe] und 50 g Polyphosphorsäure wird 5 Stunden auf 140° erhitzt. Als Zwischenprodukte entstehen in situ 4-Amino-1,2-dihydro-2-oxo-3-(N-4-(2-(1H-5-tetrazolyl)-3-thienyl)-benzyl-N-valeryl-amino)-1-(N,N-dimethylcarbamoylmethyl)-pyridin und 1,2-Dihydro-2-oxo-3-(4-(2-(1H-5-tetrazolyl)-3-thienyl-benzyl-amino)-1-(N,N-dimethylcarbamoylmethyl)-4-valerylamino-pyridin. Man kühlt ab, gießt auf Eis, macht mit Natronlauge alkalisch, arbeitet wie üblich auf und erhält 2-Butyl-3-(4-(2-(1H-5-tetrazolyl)-3-thienyl)-benzyl-4,5-dihydro-4-oxo-5-(N,N-dimethylcarbamoylmethyl)-3H-IP.

Beispiel 3

Man löst 1 g 2-Butyl-3-(4-(2-(2-triphenylmethyl-2H-5-tetrazolyl)-3-thienyl)-benzyl)-4,5-dihydro-4-oxo-5-(2-methoxycarbonylbenzyl)-3H-IP (IIIb; erhältlich durch Umsetzung von IIIa mit 2-Triphenylmethyl-5-(3-(4-brommethylphenyl-3-thienyl)-2H-tetrazol zu 2-Butyl-3-(4-(2-(2-triphenylmethyl-2H-5-tetrazolyl)-3-thienyl)-benzyl)-4,5-dihydro-4-oxo-3H-IP und Reaktion mit 2-Brommethylbenzoesäuremethylester analog Beispiel 4) in 60 ml 4n HCl in Dioxan und rührt 16 Std. bei 20°. Man dampft ein, arbeitet wie üblich auf und erhält 2-Butyl-3-(4-(2-(1H-5-tetrazolyl)-3-thienyl)-benzyl)-4,5-dihydro-4-oxo-5-(2-methoxycarbonylbenzyl)-3H-IP, Hydrat, F. 186° (Zers.).

Analog erhält man durch Reaktion der nachstehenden [2-Butyl-3-(4-(2-(2-triphenylmethyl-2H-5-tetrazolyl)-3-thienyl)-benzyl)-4,5-dihydro-4-oxo-5-R3-3H-IPen (erhältlich aus IIIb und den in Beispiel 4 (a) angegebenen Halogeniden analog Beispiel 4(a)):

-5-methyl-
-5-isopropyl-
-5-butyl-
-5-trifluormethyl-
-5-carboxymethyl-
-5-methoxycarbonylmethyl-
-5-ethoxycarbonylmethyl-
-5-phenoxycarbonylmethyl-
-5-carbamoylmethyl-
-5-(N-methyl-carbamoylmethyl)-
-5-(N,N-dimethyl-carbamoylmethyl)-
-5-(N,N-diethyl-carbamoylmethyl)-

-5-(N-phenyl-carbamoylmethyl)-
-5-(N-(2,6-dimethylphenyl)-carbamoylmethyl)-
-5-(N-methyl-N-phenyl-carbamoylmethyl)-
-5-(2-oxopropyl)-
-5-(2-oxo-3,3-dimethyl-butyl)-
-5-(2-dimethylaminoethyl)-
-5-(2-anilinoethyl)-
-5-cyclopropylmethyl-
-5-cyclobutylmethyl-
-5-cyclopentylmethyl-
-5-cyclohexylmethyl-
-5-benzyl-
-5-(2-fluorbenzyl)-
-5-(2-chlorbenzyl)-
-5-(2-carboxybenzyl)-
-5-(2-ethoxycarbonylbenzyl)-
-5-(2-nitrobenzyl)-
-5-(2-dimethylaminobenzyl)-
-5-(2-acetamidobenzyl)-
-5-(4-methoxybenzyl)-
-5-(2-thienylmethyl)-
-5-phenacyl-
-5-(2-methoxyphenacyl)-
-5-(2-oxo-2-(2-pyridyl)-ethyl)-
mit HCl in Dioxan die in Beispiel 4(b) angegebenen 2-Butyl-3-(4-(2-(1H-5-tetrazolyl)-3-thienyl)-benzyl)-4,5-dihydro-4-oxo-5-$R^3$-3H-IPe.

Beispiel 4

(a) Eine Lösung von 3,88 g 2-Butyl-3-(4-(2-cyan-3-thienyl)-benzyl)-4,5-dihydro-4-oxo-3H-IP in 35 ml DMF wird unter Rühren bei 20° mit 1,25 g K-tert.-Butylat versetzt. Nach 45 Minuten Rühren wird eine Lösung von 2,63 g 2-Brommethyl-benzoesäuremethylester in 25 ml DMF zugetropft. Man rührt noch 16 Stunden bei 20°, arbeitet wie üblich auf und erhält 2-Butyl-3-(4-(2-cyan-3-thienyl)benzyl)-4,5-dihydro-4-oxo-5-(2-methoxycarbonyl-benzyl)-3H-IP, F. 121°.

Analog erhält man die nachstehenden 2-Butyl-3-(4-(2-cyan-3-thienyl)-benzyl)-4,5-dihydro-4-oxo-5-$R^3$-3H-IPe:

| | |
|---|---|
| mit Methyliodid: | -5-methyl- |
| mit Isopropylbromid: | -5-isopropyl- |
| mit Butylbromid: | -5-butyl- |
| mit Trifluormethyliodid: | -5-trifluormethyl- |
| mit Bromessigsäure: | -5-carboxymethyl- |
| mit Bromessigsäuremethylester: | -5-methoxycarbonylmethyl- |
| mit Bromessigsäureethylester: | -5-ethoxycarbonylmethyl- |
| mit Bromessigsäurephenylester: | -5-phenoxycarbonylmethyl- |
| mit Bromacetamid: | -5-carbamoylmethyl-, F. 162-164° |
| mit N-Methyl-bromacetamid: | -5-(N-methyl-carbamoylmethyl)- |
| mit N,N-Dimethyl-chloracetamid: | -5-(N,N-dimethyl-carbamoylmethyl)-, F. 113-115° |
| mit N,N-Diethyl-chloracetamid: | -5-(N,N-diethyl-carbamoylmethyl)-, F. 126-128° |
| mit Chloracetanilid: | -5-(N-phenyl-carbamoylmethyl)- |
| mit Chloressigsäure-(2,6-dimethylanilid): | -5-(N-(2,6-dimethylphenyl)-carbamoylmethyl)- |
| mit N-Methyl-N-phenyl-chloracetamid: | -5-(N-methyl-N-phenyl-carbamoylmethyl)- |
| mit Bromaceton: | -5-(2-oxopropyl)- |
| mit 1-Brom-3,3-dimethyl-2-butanon: | -5-(2-oxo-3,3-dimethyl-butyl)- |
| mit 2-Dimethylaminoethylchlorid: | -5-(2-dimethylaminoethyl)- |
| mit 2-Anilinoethylchlorid: | -5-(2-anilinoethyl)- |
| mit Cyclopropylmethylbromid: | -5-cyclopropylmethyl- |
| mit Cyclobutylmethylchlorid: | -5-cyclobutylmethyl- |
| mit Cyclopentylmethylchlorid: | -5-cyclopentylmethyl- |

| | |
|---|---|
| mit Cyclohexylmethylchlorid: | -5-cyclohexylmethyl- |
| mit Benzylbromid: | -5-benzyl- |
| mit 2-Fluorbenzylbromid: | -5-(2-fluorbenzyl)- |
| mit 2-Chlorbenzylbromid: | -5-(2-chlorbenzyl)- |
| mit 2-Brommethyl-benzoesäure: | -5-(2-carboxybenzyl)- |
| mit 2-Brommethyl-benzoesäureethylester: | -5-(2-ethoxycarbonylbenzyl)- |
| mit 2-Nitrobenzylchlorid: | -5-(2-nitrobenzyl)- |
| mit 2-Dimethylaminobenzyl-chlorid: | -5-(2-dimethylaminobenzyl)- |
| mit 2-Acetamidobenzylchlorid: | -5-(2-acetamidobenzyl)- |
| mit 4-Methoxybenzylchlorid: | -5-(4-methoxybenzyl)- |
| mit 2-Thienylmethylchlorid: | -5-(2-thienylmethyl)- |
| mit Phenacylbromid: | -5-phenacyl- |
| mit 2-Methoxyphenacylchlorid: | -5-(2-methoxyphenacyl)- |
| mit 2-Oxo-2-(2-pyridyl)-ethyl-chlorid: | -5-(2-oxo-2-(2-pyridyl)-ethyl)-. |

(b) Ein Gemisch von 5,37 g der nach (a) erhaltenen Verbindung, 20,6 g Trimethylzinnazid und 200 ml Toluol wird 24 Stunden gekocht und dann eingedampft. Man nimmt den Rückstand in 100 ml methanolischer HCl auf, rührt 2 Stunden bei 20° und arbeitet wie üblich auf (gesättigte NaCl-Lösung/Dichlormethan). Chromatographie (Ethylacetat/Hexan 80:20) liefert 2-Butyl-3-(4-(2-(1H-5-tetrazolyl)-3-thienyl)-benzyl)-4,5-dihydro-4-oxo-5-(2-methoxycarbonylbenzyl)-3H-IP, Hydrat, F. 186 ° (Zers.).

Analog erhält man aus den unter (a) angegebenen 2-Cyan-3-thienyl-Verbindungen die nachstehenden 2-Butyl-3-(4-(2-(1H-5-tetrazolyl)-3-thienyl)-benzyl)-4,5-dihydro-4-oxo-5-$R^3$-3H-IPe:

-5-methyl-
-5-isopropyl-
-5-butyl-
-5-trifluormethyl-
-5-carboxymethyl-
-5-methoxycarbonylmethyl-
-5-ethoxycarbonylmethyl-
-5-phenoxycarbonylmethyl-
-5-carbamoylmethyl-, F. 261-262°; K-Salz, F. 292-293°
-5-(N-methyl-carbamoylmethyl)-
-5-(N,N-dimethyl-carbamoylmethyl)-, F. 216°; K-Salz, Hydrat, F. 275°
-5-(N,N-diethyl-carbamoylmethyl)-, F. 95-96°; K-Salz, F. 284-285°
-5-(N-phenyl-carbamoylmethyl)-
-5-(N-(2,6-dimethylphenyl)-carbamoylmethyl)-
-5-(N-methyl-N-phenyl-carbamoylmethyl)-
-5-(2-oxopropyl)-
-5-(2-oxo-3,3-dimethyl-butyl)-
-5-(2-dimethylaminoethyl)-
-5-(2-anilinoethyl)-
-5-cyclopropylmethyl-
-5-cyclobutylmethyl-
-5-cyclopentylmethyl-
-5-cyclohexylmethyl-
-5-benzyl-
-5-(2-fluorbenzyl)-
-5-(2-chlorbenzyl)-
-5-(2-carboxybenzyl)-
-5-(2-ethoxycarbonylbenzyl)-
-5-(2-nitrobenzyl)-
-5-(2-dimethylaminobenzyl)-
-5-(2-acetamidobenzyl)-
-5-(4-methoxybenzyl)-
-5-(2-thienylmethyl)-
-5-phenacyl-
-5-(2-methoxyphenacyl)-
-5-(2-oxo-2-(2-pyridyl)-ethyl-.

Beispiel 5

(a) Analog Beispiel 4(a) erhält man aus 2-Butyl-3-(4-(1-cyanmethyl-2-imidazolyl)-benzyl)-4,5-dihydro-4-oxo-3H-IP und 2-Brommethyl-benzoesäuremethylester das 2-Butyl-3-(4-(1-cyanmethyl-2-imidazolyl)-benzyl)-4,5-dihydro-4-oxo-5-(2-methoxycarbonyl-benzyl)-3H-IP.
Analog erhält man die nachstehenden 2-Butyl-3-(4-(1-cyanmethyl-2-imidazolyl)-benzyl)-4,5-dihydro-4-oxo-5-$R^3$-3H-IPe:

| | |
|---|---|
| mit Methyliodid: | -5-methyl- |
| mit Isopropylbromid: | -5-isopropyl- |
| mit Butylbromid: | -5-butyl- |
| mit Trifluormethyliodid: | -5-trifluormethyl- |
| mit Bromessigsäure: | -5-carboxymethyl- |
| mit Bromessigsäuremethylester: | -5-methoxycarbonylmethyl- |
| mit Bromessigsäureethylester: | -5-ethoxycarbonylmethyl- |
| mit Bromessigsäurephenylester: | -5-phenoxycarbonylmethyl- |
| mit Bromacetamid: | -5-carbamoylmethyl- |
| mit N-Methyl-bromacetamid: | -5-(N-methyl-carbamoylmethyl)- |
| mit N,N-Dimethyl-chloracetamid: | -5-(N,N-dimethyl-carbamoylmethyl)- |
| mit N,N-Diethyl-chloracetamid: | -5-(N,N-diethyl-carbamoylmethyl)- |
| mit Chloracetanilid: | -5-(N-phenyl-carbamoylmethyl)- |
| mit Chloressigsäure-(2,6-dimethylanilid): | -5-(N-(2,6-dimethylphenyl)-carbamoylmethyl) |
| mit N-Methyl-N-phenyl-chloracetamid: | -5-(N-methyl-N-phenyl-carbamoylmethyl)- |
| mit Bromaceton: | -5-(2-oxopropyl)- |
| mit 1-Brom-3,3-dimethyl-2-butanon: | -5-(2-oxo-3,3-dimethylbutyl)- |
| mit 2-Dimethylaminoethylchlorid: | -5-(2-dimethylaminoethyl)- |
| mit 2-Anilinoethylchlorid: | -5-(2-anilinoethyl)- |
| mit Cyclopropylmethylbromid: | -5-cyclopropylmethyl- |
| mit Cyclobutylmethylchlorid: | -5-cyclobutylmethyl- |
| mit Cyclopentylmethylchlorid: | -5-cyclopentylmethyl- |
| mit Cyclohexylmethylchlorid: | -5-cyclohexylmethyl- |
| mit Benzylbromid: | -5-benzyl- |
| mit 2-Fluorbenzylbromid: | -5-(2-fluorbenzyl)- |
| mit 2-Chlorbenzylbromid: | -5-(2-chlorbenzyl)- |
| mit 2-Brommethyl-benzoesäure: | -5-(2-carboxybenzyl)- |
| mit 2-Brommethyl-benzoesäureethylester: | -5-(2-ethoxycarbonylbenzyl)- |
| mit 2-Nitrobenzylchlorid: | -5-(2-nitrobenzyl)- |
| mit 2-Dimethylaminobenzylchlorid: | -5-(2-dimethylaminobenzyl)- |
| mit 2-Acetamidobenzylchlorid: | -5-(2-acetamidobenzyl)- |
| mit 4-Methoxybenzylchlorid: | -5-(4-methoxybenzyl)- |
| mit 2-Thienylmethylchlorid: | -5-(2-thienylmethyl)- |
| mit Phenacylbromid: | -5-phenacyl- |
| mit 2-Methoxyphenacylchlorid: | -5-(2-methoxyphenacyl)- |
| mit 2-Oxo-2-(2-pyridyl)-ethylchlorid: | -5-(2-oxo-2-(2-pyridyl)-ethyl)-. |

(b) Analog Beispiel 4(b) erhält man aus den unter (a) angegebenen 1-Cyanmethyl-2-imidazolyl-Verbindungen mit Trimethylzinnazid die nachstehenden 2-Butyl-3-(4-(1-(1H-5-tetrazolyl-methyl)-2-imidazolyl)-benzyl)-4,5-dihydro-4-oxo-5-$R^3$-3H-IPe:

-5-methyl-
-5-isopropyl-
-5-butyl-
-5-trifluormethyl-
-5-carboxymethyl-
-5-methoxycarbonylmethyl-
-5-ethoxycarbonylmethyl-
-5-phenoxycarbonylmethyl-
-5-carbamoylmethyl-
-5-(N-methyl-carbamoylmethyl)-
-5-(N,N-dimethyl-carbamoylmethyl)-
-5-(N,N-diethyl-carbamoylmethyl)-

-5-(N-phenyl-carbamoylmethyl)-
-5-(N-(2,6-dimethylphenyl)-carbamoylmethyl)-
-5-(N-methyl-N-phenyl-carbamoylmethyl)-
-5-(2-oxopropyl)-
-5-(2-oxo-3,3-dimethyl-butyl)-
-5-(2-dimethylaminoethyl)-
-5-(2-anilinoethyl)-
-5-cyclopropylmethyl-
-5-cyclobutylmethyl-
-5-cyclopentylmethyl-
-5-cyclohexylmethyl-
-5-benzyl-
-5-(2-fluorbenzyl)-
-5-(2-chlorbenzyl)-
-5-(2-carboxybenzyl)-
-5-(2-ethoxycarbonylbenzyl)-
-5-(2-nitrobenzyl)-
-5-(2-dimethylaminobenzyl)-
-5-(2-acetamidobenzyl)-
-5-(4-methoxybenzyl)-
-5-(2-thienylmethyl)-
-5-phenacyl-
-5-(2-methoxyphenacyl)-
-5-(2-oxo-2-(2-pyridyl)-ethyl)-.

Beispiel 6

(a) Analog Beispiel 4(a) erhält man aus 2-Butyl-3-(4-(1-cyanmethyl-4,5-dichlor-2-imidazolyl)-benzyl)-4,5-dihydro-4-oxo-3H-IP und 2-Brommethyl-benzoesäuremethylester das 2-Butyl-3-(4-(1-cyanmethyl-4,5-dichlor-2-imidazolyl)-benzyl)-4,5-dihydro-4-oxo-5-(2-methoxycarbonyl-benzyl)-3H-IP.

Analog erhält man die nachstehenden 2-Butyl-3-(4-(1-cyanmethyl-4,5-dichlor-2-imidazolyl)-benzyl)-4,5-dihydro-4-oxo-5-$R^3$-3H-IPe:

| | |
|---|---|
| mit Methyliodid: | -5-methyl- |
| mit Isopropylbromid: | -5-isopropyl- |
| mit Butylbromid: | -5-butyl- |
| mit Trifluormethyliodid: | -5-trifluormethyl- |
| mit Bromessigsäure: | -5-carboxymethyl- |
| mit Bromessigsäuremethylester: | -5-methoxycarbonylmethyl- |
| mit Bromessigsäureethylester: | -5-ethoxycarbonylmethyl- |
| mit Bromessigsäurephenylester: | -5-phenoxycarbonylmethyl- |
| mit Bromacetamid: | -5-carbamoylmethyl- |
| mit N-Methyl-bromacetamid: | -5-(N-methyl-carbamoylmethyl)- |
| mit N,N-Dimethyl-chloracetamid: | -5-(N,N-dimethyl-carbamoylmethyl)- |
| mit N,N-Diethyl-chloracetamid: | -5-(N,N-diethyl-carbamoylmethyl)- |
| mit Chloracetanilid: | -5-(N-phenyl-carbamoylmethyl)- |
| mit Chloressigsäure-(2,6-dimethylanilid): | -5-(N-(2,6-dimethylphenyl)-carbamoylmethyl)- |
| mit N-Methyl-N-phenyl-chloracetamid: | -5-(N-methyl-N-phenyl-carbamoylmethyl)- |
| mit Bromaceton: | -5-(2-oxopropyl)- |
| mit 1-Brom-3,3-dimethyl-2-butanon: | -5-(2-oxo-3,3-dimethyl-butyl)- |
| mit 2-Dimethylaminoethylchlorid: | -5-(2-dimethylaminoethyl)- |
| mit 2-Anilinoethylchlorid: | -5-(2-anilinoethyl)- |
| mit Cyclopropylmethylbromid: | -5-cyclopropylmethyl- |
| mit Cyclobutylmethylchlorid: | -5-cyclobutylmethyl- |
| mit Cyclopentylmethylchlorid: | -5-cyclopentylmethyl- |
| mit Cyclohexylmethylchlorid: | -5-cyclohexylmethyl- |
| mit Benzylbromid: | -5-benzyl- |
| mit 2-Fluorbenzylbromid: | -5-(2-fluorbenzyl)- |
| mit 2-Chlorbenzylbromid: | -5-(2-chlorbenzyl)- |

| | |
|---|---|
| mit 2-Brommethyl-benzoesäure: | -5-(2-carboxybenzyl)- |
| mit 2-Brommethyl-benzoesäureethylester: | -5-(2-ethoxycarbonylbenzyl)- |
| mit 2-Nitrobenzylchlorid: | -5-(2-nitrobenzyl)- |
| mit 2-Dimethylaminobenzylchlorid: | -5-(2-dimethylaminobenzyl)- |
| mit 2-Acetamidobenzylchlorid: | -5-(2-acetamidobenzyl)- |
| mit 4-Methoxybenzylchlorid: | -5-(4-methoxybenzyl)- |
| mit 2-Thienylmethylchlorid: | -5-(2-thienylmethyl)- |
| mit Phenacylbromid: | -5-phenacyl- |
| mit 2-Methoxyphenacylchlorid: | -5-(2-methoxyphenacyl)- |
| mit 2-Oxo-2-(2-pyridyl)-ethyl-chlorid: | -5-(2-oxo-2-(2-pyridyl)-ethyl)-. |

(b) Analog Beispiel 4(b) erhält man aus den unter (a) angegebenen 1-Cyanmethyl-4,5-dichlor-2-imidazolyl-Verbindungen mit Trimethylzinnazid die nachstehenden 2-Butyl-3-(4-(1-(1H-5-tetrazolyl-methyl)-4,5-dichlor-2-imidazolyl)-benzyl)-4,5-dihydro-4-oxo-5-$R^3$-3H-IPe:

-5-methyl-
-5-isopropyl-
-5-butyl-
-5-trifluormethyl-
-5-carboxymethyl-
-5-methoxycarbonylmethyl-
-5-ethoxycarbonylmethyl-
-5-phenoxycarbonylmethyl-
-5-carbamoylmethyl-
-5-(N-methyl-carbamoylmethyl)-
-5-(N,N-dimethyl-carbamoylmethyl)-
-5-(N,N-diethyl-carbamoylmethyl)-
-5-(N-phenyl-carbamoylmethyl)-
-5-(N-(2,6-dimethylphenyl)-carbamoylmethyl)-
-5-(N-methyl-N-phenyl-carbamoylmethyl)-
-5-(2-oxopropyl)-
-5-(2-oxo-3,3-dimethyl-butyl)-
-5-(2-dimethylaminoethyl)-
-5-(2-anilinoethyl)-
-5-cyclopropylmethyl-
-5-cyclobutylmethyl-
-5-cyclopentylmethyl-
-5-cyclohexylmethyl-
-5-benzyl-
-5-(2-fluorbenzyl)-
-5-(2-chlorbenzyl)-
-5-(2-carboxybenzyl)-
-5-(2-ethoxycarbonylbenzyl)-
-5-(2-nitrobenzyl)-
-5-(2-dimethylaminobenzyl)-
-5-(2-acetamidobenzyl)-
-5-(4-methoxybenzyl)-
-5-(2-thienylmethyl)-
-5-phenacyl-
-5-(2-methoxyphenacyl)-
-5-(2-oxo-2-(2-pyridyl)-ethyl)-.

Beispiel 7

(a) Analog Beispiel 4(a) erhält man aus 2-Butyl-3-(4-(1-ethoxycarbonylmethyl-2-imidazolyl)-benzyl)-4,5-dihydro-4-oxo-3H-IP und 2-Brommethyl-benzoesäuremethylester das 2-Butyl-3-(4-(1-ethoxycarbonylmethyl-2-imidazolyl)-benzyl)-4,5-dihydro-4-oxo-5-(2-methoxycarbonyl-benzyl)-3H-IP.

Analog erhält man die nachstehenden 2-Butyl-3-(4-(1-ethoxycarbonylmethyl-2-imidazolyl)-benzyl)-4,5-dihydro-4-oxo-5-$R^3$-3H-IPe:

| | |
|---|---|
| mit Methyliodid: | -5-methyl- |

| | |
|---|---|
| mit Isopropylbromid: | -5-isopropyl- |
| mit Butylbromid: | -5-butyl- |
| mit Trifluormethyliodid: | -5-trifluormethyl- |
| mit Bromessigsäure: | -5-carboxymethyl- |
| mit Bromessigsäuremethylester: | -5-methoxycarbonylmethyl- |
| mit Bromessigsäureethylester: | -5-ethoxycarbonylmethyl- |
| mit Bromessigsäurephenylester: | -5-phenoxycarbonylmethyl- |
| mit Bromacetamid: | -5-carbamoylmethyl- |
| mit N-Methyl-bromacetamid: | -5-(N-methyl-carbamoylmethyl)- |
| mit N,N-Dimethyl-chloracetamid: | -5-(N,N-dimethyl-carbamoylmethyl)- |
| mit N,N-Diethyl-chloracetamid: | -5-(N,N-diethyl-carbamoylmethyl)- |
| mit Chloracetanilid: | -5-(N-phenyl-carbamoylmethyl)- |
| mit Chloressigsäure-(2,6-dimethylanilid): | -5-(N-(2,6-dimethylphenyl)-carbamoylmethyl)- |
| mit N-Methyl-N-phenyl-chloracetamid: | -5-(N-methyl-N-phenyl-carbamoylmethyl)- |
| mit Bromaceton: | -5-(2-oxopropyl)- |
| mit 1-Brom-3,3-dimethyl-2-butanon: | -5-(2-oxo-3,3-dimethyl-butyl)- |
| mit 2-Dimethylaminoethylchlorid: | -5-(2-dimethylaminoethyl)- |
| mit 2-Anilinoethylchlorid: | -5-(2-anilinoethyl)- |
| mit Cyclopropylmethylbromid: | -5-cyclopropylmethyl- |
| mit Cyclobutylmethylchlorid: | -5-cyclobutylmethyl- |
| mit Cyclopentylmethylchlorid: | -5-cyclopentylmethyl- |
| mit Cyclohexylmethylchlorid: | -5-cyclohexylmethyl- |
| mit Benzylbromid: | -5-benzyl- |
| mit 2-Fluorbenzylbromid: | -5-(2-fluorbenzyl)- |
| mit 2-Chlorbenzylbromid: | -5-(2-chlorbenzyl)- |
| mit 2-Brommethyl-benzoesäure: | -5-(2-carboxybenzyl)- |
| mit 2-Brommethyl-bensoesäureethylester: | -5-(2-ethoxycarbonylbenzyl)- |
| mit 2-Nitrobenzylchlorid: | -5-(2-nitrobenzyl)- |
| mit 2-Dimethylaminobenzylchlorid: | -5-(2-dimethylamino-benzyl)- |
| mit 2-Acetamidobenzylchlorid: | -5-(2-acetamidobenzyl)- |
| mit 4-Methoxybenzylchlorid: | -5-(4-methoxybenzyl)- |
| mit 2-Thienylmethylchlorid: | -5-(2-thienylmethyl)- |
| mit Phenacylbromid: | -5-phenacyl- |
| mit 2-Methoxyphenacylchlorid: | -5-(2-methoxyphenacyl)- |
| mit 2-Oxo-2-(2-pyridyl)-ethyl-chlorid: | -5-(2-oxo-2-(2-pyridyl)-ethyl)-. |

(b) Ein Gemisch von 1 g 2-Butyl-3-(4-(1-ethoxycarbonyl-methyl-2-imidazolyl)-benzyl)-4,5-dihydro-4-oxo-5-(2-methoxycarbonyl-benzyl)-3H-IP, 12 ml wässeriger 2n NaOH-Lösung und 48 ml Methanol wird 2 Stunden gekocht, dann eingedampft. Man arbeitet wie üblich auf (wässerige Salzsäure bis pH 3/Dichlormethan) und erhält 2-Butyl-3-(4-(1-carboxymethyl-2-imidazolyl)-benzyl)-4,5-dihydro-4-oxo-5-(2-carboxybenzyl)-3H-IP.

Analog erhält man durch Verseifung der vorstehend unter (a) angegebenen Ester die nachstehenden 2-Butyl-3-(4-(1-carboxymethyl-2-imidazolyl)-benzyl)-4,5-dihydro-4-oxo-5-$R^3$-3H-IPe:

-5-methyl-
-5-isopropyl-
-5-butyl-
-5-trifluormethyl-
-5-carboxymethyl-
-5-methoxycarbonylmethyl-
-5-ethoxycarbonylmethyl-
-5-phenoxycarbonylmethyl-
-5-carbamoylmethyl-
-5-(N-methyl-carbamoylmethyl)-
-5-(N,N-dimethyl-carbamoylmethyl)-
-5-(N,N-diethyl-carbamoylmethyl)-
-5-(N-phenyl-carbamoylmethyl)-
-5-(N-(2,6-dimethylphenyl)-carbamoylmethyl)-
-5-(N-methyl-N-phenyl-carbamoylmethyl)-
-5-(2-oxopropyl)-

-5-(2-oxo-3,3-dimethyl-butyl)-
-5-(2-dimethylaminoethyl)-
-5-(2-anilinoethyl)-
-5-cyclopropylmethyl-
-5-cyclobutylmethyl-
-5-cyclopentylmethyl-
-5-cyclohexylmethyl-
-5-benzyl-
-5-(2-fluorbenzyl)-
-5-(2-chlorbenzyl)-
-5-(2-carboxybenzyl)-
-5-(2-ethoxycarbonylbenzyl)-
-5-(2-nitrobenzyl)-
-5-(2-dimethylaminobenzyl)-
-5-(2-acetamidobenzyl)-
-5-(4-methoxybenzyl)-
-5-(2-thienylmethyl)-
-5-phenacyl-
-5-(2-methoxyphenacyl)-
-5-(2-oxo-2-(2-pyridyl)-ethyl)-.

Beispiel 8

(a) Analog Beispiel 4(a) erhält man aus 2-Butyl-3-(4-(1-ethoxycarbonylmethyl-4,5-dichlor-2-imidazolyl)-benzyl)-4,5-dihydro-4-oxo-3H-IP und 2-Brommethyl-benzoesäuremethylester das 2-Butyl-3-(4-(1-ethoxycarbonylmethyl-4,5-dichlor-2-imidazolyl)-benzyl)-4,5-dihydro-4-oxo-5-(2-methoxycarbonyl-benzyl)-3H-IP.

Analog erhält man die nachstehenden 2-Butyl-3-(4-(1-ethoxycarbonylmethyl-4,5-dichlor-2-imidazolyl)-benzyl)-4,5-dihydro-4-oxo-5-$R^3$-3H-IPe:

| | |
|---|---|
| mit Methyliodid: | -5-methyl- |
| mit Isopropylbromid: | -5-isopropyl- |
| mit Butylbromid: | -5-butyl- |
| mit Trifluormethyliodid: | -5-trifluormethyl- |
| mit Bromessigsäure: | -5-carboxymethyl- |
| mit Bromessigsäuremethylester: | -5-methoxycarbonylmethyl- |
| mit Bromessigsäureethylester; | -5-ethoxycarbonylmethyl- |
| mit Bromessigsäurephenylester; | -5-phenoxycarbonylmethyl- |
| mit Bromacetamid: | -5-carbamoylmethyl- |
| mit N-Methyl-bromacetamid: | -5-(N-methyl-carbamoylmethyl)- |
| mit N,N-Dimethyl-chloracetamid: | -5-(N,N-dimethyl-carbamoylmethyl)- |
| mit N,N-Diethyl-chloracetamid: | -5-(N,N-diethyl-carbamoylmethyl)- |
| mit Chloracetanilid: | -5-(N-phenyl-carbamoylmethyl)- |
| mit Chloressigsäure-(2,6-dimethylanilid): | -5-(N-(2,6-dimethylphenyl)-carbamoylmethyl)- |
| mit N-Methyl-N-phenyl-chloracetamid: | -5-(N-methyl-N-phenyl-carbamoylmethyl)- |
| mit Bromaceton: | -5-(2-oxopropyl)- |
| mit 1-Brom-3,3-dimethyl-2-butanon: | -5-(2-oxo-3,3-dimethyl-butyl)- |
| mit 2-Dimethylaminoethylchlorid: | -5-(2-dimethylaminoethyl)- |
| mit 2-Anilinoethylchlorid: | -5-(2-anilinoethyl)- |
| mit Cyclopropylmethylbromid: | -5-cyclopropylmethyl- |
| mit Cyclobutylmethylchlorid: | -5-cyclobutylmethyl- |
| mit Cyclopentylmethylchlorid: | -5-cyclopentylmethyl- |
| mit Cyclohexylmethylchlorid: | -5-cyclohexylmethyl- |
| mit Benzylbromid: | -5-benzyl- |
| mit 2-Fluorbenzylbromid: | -5-(2-fluorbenzyl)- |
| mit 2-Chlorbenzylbromid: | -5-(2-chlorbenzyl)- |
| mit 2-Brommethyl-benzoesäure: | -5-(2-carboxybenzyl)- |
| mit 2-Brommethyl-benzoesäureethylester: | -5-(2-ethoxycarbonylbenzyl)- |
| mit 2-Nitrobenzylchlorid: | -5-(2-nitrobenzyl)- |
| mit 2-Dimethylaminobenzylchlorid: | -5-(2-dimethylaminobenzyl)- |

| | |
|---|---|
| mit 2-Acetamidobenzylchlorid: | -5-(2-acetamidobenzyl)- |
| mit 4-Methoxybenzylchlorid: | -5-(4-methoxybenzyl)- |
| mit 2-Thienylmethylchlorid: | -5-(2-thienylmethyl)- |
| mit Phenacylbromid: | -5-phenacyl- |
| mit 2-Methoxyphenacylchlorid: | -5-(2-methoxyphenacyl)- |
| mit 2-Oxo-2-(2-pyridyl)-ethyl-chlorid: | -5-(2-oxo-2-(2-pyridyl)-ethyl)-. |

(b) Analog Beispiel 7(b) erhält man durch Verseifung von 2-Butyl-3-(4-(1-ethoxycarbonylmethyl-4,5-dichlor-2-imidazolyl)-benzyl)-4,5-dihydro-4-oxo-5-(2-methoxycarbonylbenzyl)-3H-IP das 2-Butyl-3-(4-(1-carboxymethyl-4,5-dichlor-2-imidazolyl)-benzyl)-4,5-dihydro-4-oxo-5-(2-carboxybenzyl)-3H-IP.

Analog erhält man durch Verseifung der vorstehend unter (a) angegebenen Ester die nachstehenden 2-Butyl-3-(4-(1-carboxymethyl-4,5-dichlor-2-imidazolyl)-benzyl)-4,5-dihydro-4-oxo-5-$R^3$-3H-IPe:

-5-methyl-
-5-isopropyl-
-5-butyl-
-5-trifluormethyl-
-5-carboxymethyl-
-5-methoxycarbonylmethyl-
-5-ethoxycarbonylmethyl-
-5-phenoxycarbonylmethyl-
-5-carbamoylmethyl-
-5-(N-methyl-carbamoylmethyl)-
-5-(N,N-dimethyl-carbamoylmethyl)-
-5-(N,N-diethyl-carbamoylmethyl)-
-5-(N-phenyl-carbamoylmethyl)-
-5-(N-(2,6-dimethylphenyl)-carbamoylmethyl)-
-5-(N-methyl-N-phenyl-carbamoylmethyl)-
-5-(2-oxopropyl)-
-5-(2-oxo-3,3-dimethyl-butyl)-
-5-(2-dimethylaminoethyl)-
-5-(2-anilinoethyl)-
-5-cyclopropylmethyl-
-5-cyclobutylmethyl-
-5-cyclopentylmethyl-
-5-cyclohexylmethyl-
-5-benzyl-
-5-(2-fluorbenzyl)-
-5-(2-chlorbenzyl)-
-5-(2-carboxybenzyl)-
-5-(2-ethoxycarbonylbenzyl)-
-5-(2-nitrobenzyl)-
-5-(2-dimethylaminobenzyl)-
-5-(2-acetamidobenzyl)-
-5-(4-methoxybenzyl)-
-5-(2-thienylmethyl)-
-5-phenacyl-
-5-(2-methoxyphenacyl)-
-5-(2-oxo-2-(2-pyridyl)-ethyl)-.

Beispiel 9

Analog Beispiel 7(b) erhält man durch Verseifung von 2-Bintyl-3-(4-(2-(1H-5-tetrazolyl)-3-thienyl)-benzyl)-4,5-dihydro-4-oxo-5-(2-methoxycarbonylbenzyl)-3H-IP das 2-Butyl-3-(4-(2-(1H-5-tetrazolyl)-3-thienyl)-benzyl)-4,5-dihydro-4-oxo-5-(2-carboxybenzyl)-3H-IP.

Analog erhält man durch Verseifung der entsprechenden in den Beispielen 4-6 angegebenen Methyl- oder Ethylester die dort ebenfalls angegebenen Carbonsäuren.

Beispiel 10

Eine Lösung von 1 g 2-Butyl-3-(4-(2-(1H-5-tetrazolyl)-3-thienyl)-benzyl)-4,5-dihydro-4-oxo-5-(2-nitro-benzyl)-3H-IP in 20 ml Methanol wir an 0,3 g 5%iger Pd-Kohle bei 20° und Normaldruck bis zur Aufnahme der berechneten $H_2$-Menge hydriert. Man filtriert den Katalysator ab, dampft ein und erhält 2-Butyl-3-(4-(2-(1H-5-tetrazolyl)-3-thienyl)-benzyl)-4,5-dihydro-4-oxo-5-(2-aminobenzyl)-3H-IP.

Beispiel 11

Eine Lösung von 1 g 2-Butyl-3-(4-(2-(1H-5-tetrazolyl)-3-thienyl)-benzyl)-4,5-dihydro-4-oxo-5-(6-BOC-amino-hexyl)-3H-IP in 20 ml Dichlormethan und 20 ml Trifluoressigsäure wird 1 Std. bei 20° gerührt, eingedampft und wie üblich aufgearbeitet Man erhält 2-Butyl-3-(4-(2-(1H-5-tetrazolyl)-3-thienyl)-benzyl)-4,5-dihydro-4-oxo-5-(6-aminohexyl)-3H-IP.

Die nachstehenden Beispiele betreffen pharmazeutische Zubereitungen, die Wirkstoffe der Formel I oder ihre Salze enthalten.

Beispiel A: Tabletten und Dragees

In üblicher Weise werden Tabletten folgender Zusammensetzung gepreßt, die bei Bedarf mit einer üblichen Drageedecke auf Sucrosegrundlage überzogen werden:

| | |
|---|---|
| Wirkstoff der Formel I | 100 mg |
| Mikrokristalline Cellulose | 278,8 mg |
| Lactose | 110 mg |
| Maisstärke | 11 mg |
| Magnesiumstearat | 5 mg |
| Feinteiliges Siliciumdioxid | 0,2 mg |

Beispiel B: Hartgelatine-Kapseln

Übliche zweiteilige Hartgelatine-Kapseln werden jeweils gefüllt mit

| | |
|---|---|
| Wirkstoff der Formel I | 100 mg |
| Lactose | 150 mg |
| Cellulose | 50 mg |
| Magnesiumstearat | 6 mg |

Beispiel C: Weichgelatine-Kapseln

Übliche Weichgelatine-Kapseln werden mit einem Gemisch aus jeweils 50 mg Wirkstoff und 250 mg Olivenöl gefüllt.

Beispiel D: Ampullen

Eine Lösung von 200 g Wirkstoff in 2 kg 1,2-Propandiol wird mit Wasser auf 10 l aufgefüllt und in Ampullen gefüllt, so daß jede Ampulle 20 mg Wirkstoff enthält.

Beispiel E: Wässerige Suspension für orale Applikation

Eine wässerige Suspension des Wirkstoffs wird in üblicher Weise hergestellt. Die Einheitsdosis (5 ml) enthält 100 mg Wirkstoff, 100 mg Na-Carboxymethylcellulose, 5 mg Na-Benzoat und 100 mg Sorbit.

## Patentansprüche

**1.** Imidazopyridinderivate der Formel I

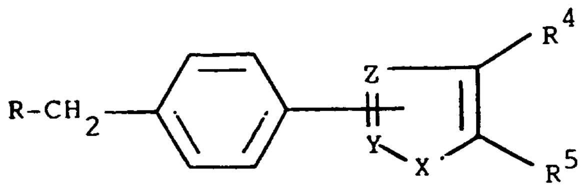

I

worin

R

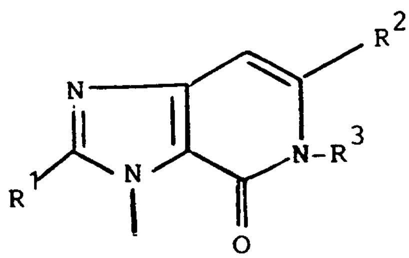

X — O, S oder $NR^6$,

-Y=Z- — $-CR^7=C-$, $-C=CR^7-$, $-N=C-$ oder $-C=N-$,

| | |
|---|---|
| $R^1$ | A, Alkenyl oder Alkinyl mit jeweils bis zu 6 C-Atomen, Cycloalkyl mit 3-7 C-Atomen, OA oder SA, |
| $R^2$ | H oder Hal, |
| $R^3$ | H, $R^8$ oder $-C_nH_{2n}-R^9$, |
| $R^4$ und $R^5$ | jeweils H, A oder Hal, |
| $R^6$ | H oder $-C_mH_{2m}-R^{10}$, |
| $R^7$ und $R^{10}$ | jeweils CN, $COOR^{11}$ oder 1H-5-Tetrazolyl, |
| $R^8$ | Alkyl mit 1-6 C-Atomen, worin auch ein oder mehrere H-Atom(e) durch F ersetzt sein kann (können), |
| $R^9$ | $COOR^{12}$, $CONR^{12}R^{13}$, COA, $NR^{12}R^{13}$, Cycloalkyl mit 3-7 C-Atomen, Ar, Het, COAr oder COHet, |
| $R^{11}$, $R^{12}$ und $R^{13}$ | jeweils H, A oder Ar, |
| A | Alkyl mit 1-6 C-Atomen, |
| Ar | eine unsubstituierte oder eine durch $R^8$, OH, $OR^8$, COOH, COOA, CN, $NO_2$, $NH_2$, $NHCOR^8$, $NHSO_2R^8$, Hal oder 1H-5-Tetrazolyl mono- oder disubstituierte Phenylgruppe, |
| Het | einen fünf- oder sechsgliedrigen heteroaromatischen Rest mit 1 bis 3 N-, O- und/oder S-Atomen, der auch ein- oder zweifach durch A substituiert und/oder mit einem Benzol- oder Pyridinring kondensiert sein kann, |
| Hal | F, Cl, Br oder I und |
| m und n | jeweils 1, 2, 3, 4, 5 oder 6 bedeuten, |

sowie ihre Salze.

**2.**

a) 2-Butyl-3-(4-(2-cyan-3-thienyl)-benzyl)-4,5-dihydro-4-oxo-3H-imidazo[4,5-c]pyridin;
b) 2-Butyl-3-(4-(2-cyan-3-thienyl)-benzyl)-4,5-dihydro-4-oxo-5-(2-methoxycarbonyl-benzyl)-3H-imidazo[4,5-c]-pyridin;
c) 2-Butyl-3-(4-(2-(1H-5-tetrazolyl)-3-thienyl)-benzyl)-4,5-dihydro-4-oxo-5-(2-methoxycarbonyl-benzyl)-3H-imidazo[4,5-c]pyridin.

**3.** Verfahren zur Herstellung von Imidazopyridinen der Formel I nach Anspruch 1 sowie ihrer Salze, dadurch gekennzeichnet, daß man
(a) eine Verbindung der Formel II

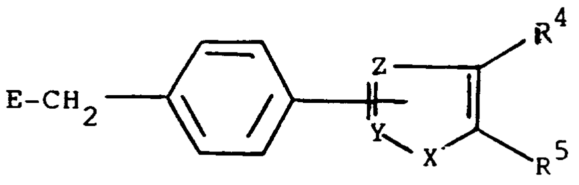

II

worin
E Cl, Br, I oder eine freie oder reaktionsfähig funktionell angewandelte OH-Gruppe bedeutet und
X, -Y=Z-, $R^4$ und $R^5$ die in Anspruch 1 angegebene Bedeutung haben,
mit einer Verbindung der Formel III

H-R III

worin
R die in Anspruch 1 angegebene Bedeutung hat,
umsetzt
oder
(b) eine Verbindung der Formel IV

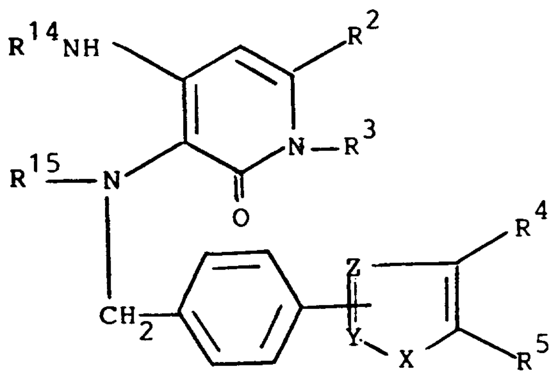

IV

worin
$R^{14}$ $R^1$-CO oder H und
$R^{15}$ H (falls $R^{14}$ $R^1$-CO ist) oder $R^1$-CO (falls $R^{14}$ H ist)
bedeuten
und
X, -Y=Z-, $R^1$, $R^2$, $R^3$, $R^4$ und $R^5$ die in Anspruch 1 angegebenen Bedeutungen haben,
mit einem cyclisierenden Mittel behandelt,
oder
(c) eine Verbindung der Formel I aus einem ihrer funktionellen Derivate durch Behandeln mit einem solvolysierenden oder hydrogenolysierenden Mittel in Freiheit setzt,

und/oder daß man in einer Verbindung der Formel I einen oder mehrere Rest(e) R, X und/oder -Y=Z- in einen oder mehrere andere Reste R, X und/oder -Y=Z- umwandelt und/oder eine Base oder Säure der Formel I in eines ihrer Salze umwandelt.

**4.** Verfahren zur Herstellung pharmazeutischer Zubereitungen, dadurch gekennzeichnet, daß man eine Verbindung der Formel I nach Anspruch 1 und/oder eines ihrer physiologisch unbedenklichen Säureadditionssalze zusammen mit mindestens einem festen, flüssigen oder halbflüssigen Träger- und Hilfsstoff in eine geeignete Dosierungsform bringt.

**5.** Pharmazeutische Zubereitung, gekennzeichnet durch einen Gehalt an mindestens einer Verbindung der Formel I nach Anspruch 1 und/oder einem ihrer physiologisch unbedenklichen Säureadditionssalze.

**6.** Verbindung der Formel I nach Anspruch 1 und ihre physiologisch unbedenklichen Säureadditionssalze zur Bekämpfung von Krankheiten.

**7.** Verwendung von Verbindungen der Formel I nach Anspruch 1 und/oder ihrer physiologisch unbedenklichen Säureadditionssalze zur Herstellung eines Arzneimittels.

**8.** Verwendung von Verbindungen der Formel I nach Anspruch 1 und/oder ihrer physiologisch unbedenklichen Säureadditionssalze bei der Bekämpfung von Krankheiten.